# EUROPEAN PATENT SPECIFICATION

(11) **EP 0 474 733 B1**
(45) Date of publication and mention of the grant of the patent: **31.08.1994**
(21) Application number: 90908860.1
(22) Date of filing: 31.05.1990
(51) Int. Cl.: C07C 275/28

(54) **ANTIHYPERLIPIDEMIC AND ANTIATHEROSCLEROTIC UREA COMPOUNDS**
ANTIHYPERLIPIDEMISCHE UND ANTIARTHEROSKLEROTISCHE HARNSTOFFVERBINDUNGEN
COMPOSES D'UREE ANTIHYPERLIPIDIMIQUE ET ANTIATHEROSCLEROTIQUE

(30) Priority: 01.06.1989 US 359830
(43) Date of publication of application: 18.03.1992
(73) Proprietor: WARNER-LAMBERT COMPANY, Ann Arbor, Michigan 48105 (US)
(72) Inventor: TRIVEDI, Bharat, Kalidas, Farmington Hill Hills MI 48335 (US)
(74) Representative: Mansmann, Ivo
(86) International application number: PCT/US90/03055
(87) International publication number: WO 90/15048

(56) References cited:
- EP-A- 0 293 880

## Description

This invention relates to chemical compounds having pharmacological activity, to pharmaceutical compositions which include these compounds, and to a pharmaceutical method of treatment. More particularly, this invention concerns certain substituted urea compounds which inhibit the enzyme acyl-coenzyme A:cholesterol acyl-transferase (ACAT), pharmaceutical compositions containing these compounds, and a method of inhibiting intestinal absorption of cholesterol or of regulating cholesterol.

In recent years the role which elevated plasma levels of cholesterol plays in pathological conditions in man has received much attention. Deposits of cholesterol in the vascular system have been indicated as causative of a variety of pathological conditions including coronary heart disease.

Initially, studies of this problem were directed toward finding therapeutic agents which would be effective in lowering total serum cholesterol levels. It is now known that cholesterol is transported in the blood in the form of complex particles consisting of a core of cholesterol esters plus triglycerides and an exterior consisting primarily of phospholipids and a variety of types of protein which are recognized by specific receptors. For example, cholesterol is carried to the sites of deposit in blood vessels in the form of low density lipoprotein cholesterol (LDL cholesterol) and away from such sites of deposit by high density lipo-protein cholesterol (HDL cholesterol).

Following these discoveries, the search for therapeutic agents which control serum cholesterol turned to finding compounds which are more selective in their action; that is, agents which are effective in elevating the blood serum levels of HDL cholesterol and/or lowering the levels of LDL cholesterol. While such agents are effective in moderating the levels of serum cholesterol, they have little or no effect on controlling the initial absorption of dietary cholesterol in the body through the intestinal wall.

In intestinal mucosal cells, dietary cholesterol is absorbed as free cholesterol which must be esterified by the action of the enzyme acyl-CoA: cholesterol acyltransferase (ACAT) before it can be packaged into the chylomicrons which are then released into the blood stream. Thus, therapeutic agents which effectively inhibit the action of ACAT prevent the intestinal absorption of dietary cholesterol into the blood stream or the reabsorption of cholesterol which has been previously released into the intestine through the body's own regulatory action.

The compounds of the present invention wherein n is zero and R₁ and R₂ are alkyl having from one to four carbon atoms are disclosed generically in European Patent Application Number 88108816.5, which was published December 7, 1988 (publication number 293,880). The following references are more remotely related:

U.S. Patent No. 4,397,868 (De Vries) discloses phenylurea compounds useful in reducing cholesterol wherein one of the nitrogen atoms is disubstituted with, e.g., aliphatic, alicyclic or aromatic groups.

U.S. Patent 4,410,697 (Torok) discloses an improved process for preparing phenylurea compounds which are useful as herbicides, rodenticides, bacteriostatic agents, coccidiostatic agents and antiseptic agents. The non-aniline nitrogen in these compounds can be mono- or di-substituted with alkyl, cycloalkyl, alkoxy or phenyl which groups may be further substituted.

U.S. 4,623,662 (De Vries) discloses phenylurea and phenylthiourea compounds useful in treating atherosclerosis wherein the non-aniline nitrogen is disubstituted with aliphatic, cycloalkylalkyl, aralykyl groups wherein the aryl moiety may be substituted and wherein the aniline phenyl moiety is substituted with a wide variety of groups.

The present invention provides the following compounds having ACAT inhibitory activity having the structure 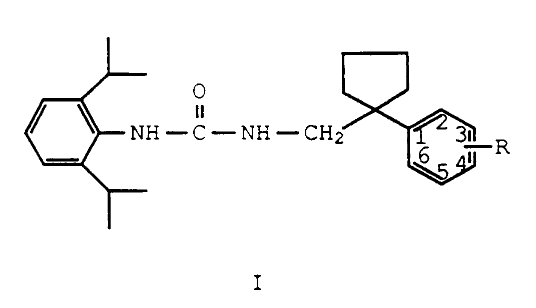
wherein R is attached to the 3- or 4-position of the phenyl ring and is the group: 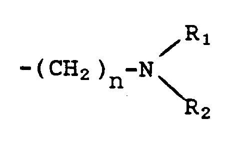
wherein n is zero, one or two, and
wherein R₁ and R₂ are straight or branched alkyl having from one to four carbon atoms or alkyl having from one to four carbon atoms wherein the terminal carbon atom is substituted with an OR₃ group wherein R₃ is hydrogen or alkyl having from one to four carbon atoms.

### DETAILED DESCRIPTION

The compounds of the present invention form a particularly active group of substituted ureas compound having potent activity as inhibitors of the enzyme acyl CoA: cholesterol acyltransferase (ACAT). The most preferred compounds of the present invention are those wherein R₁ and R₂ are methyl, and n is zero or one. Compounds wherein n is one are more preferred.

Pharmaceutically acceptable acid addition salts are included within the scope of this invention.

While the acid addition salts may vary from the free base form of the compounds in certain properties such as melting point and solubility, they are considered equivalent to the free base forms for the purposes of this invention.

The acid addition salts may be generated from the free base forms of the compounds by reaction of the latter with one equivalent of a suitable nontoxic, pharmaceutically acceptable acid, followed by evaporation of the solvent employed for the reaction and recrystallization of the salt, if required. The free base may be recovered from the acid addition salt by reaction of the salt with a water solution of the salt with a suitable base such as sodium carbonate, sodium bicarbonate, potassium carbonate, sodium hydroxide, and the like.

Suitable acids for forming acid addition salts of the compounds of this invention include, but are not necessarily limited to acetic, benzoic, benzenesulfonic, tartaric, hydrobromic, hydrochloric, citric, fumaric, gluconic, glucuronic, glutamic, lactic, malic, maleic, methanesulfonic, pamoic, salicylic, stearic, succinic, sulfuric, and tartaric acids. The class of acids suitable for the formation of nontoxic, pharmaceutically acceptable salts is well known to practitioners of the pharmaceutical formulation arts. (See, for example, Stephen N. Berge, et al, J. Pharm. Sciences, 66:1-19 (1977).

The compounds of this invention may exist in unsolvated as well as solvated forms with pharmaceutically acceptable solvents such as water, ethanol and the like. In general, the solvated forms are considered equivalent to the unsolvated forms for the purposes of this invention.

The compounds of general Formula I wherein n is zero are prepared by the procedure set forth in Chart I. The reaction is generally carried out in a polar aprotic organic solvent such as ethyl acetate, at any temperature between room temperature and the boiling point of the solvent, with room temperature being preferred. The reaction is allowed to proceed until analysis of the mixture by a means such as chromatography indicates that the reaction is substantially complete. Reaction times may vary between about two hours to about 24 hours, depending upon the particular reagents and reaction temperature employed. The starting isocyanate compound is known in the art.

The compounds of general Formula I wherein n is one are prepared by the procedure set forth in Chart II. The ester derivative (4) is hydrolysed to the acid derivative (5) using in NaOH in methanol. The reaction is carried out at reflux temperature for 20 hours. The acid (5) is then reduced to the alcohol (6) using boron methyl sulfide in THF. The reaction is initially refluxed for 4 hours then stirred at room temperature for 20 hours. The alcohol (6) is converted to the bromo derivative (7) by reacting it with phosphorus tribromide in pyridine and toluene. It is preferred that the reaction is carried out initially at 0°C (up to 5 hours) followed by stirring at room temperature for additional 72 hours. The bromo derivative (7) is then reacted with dimethylamine in methanol at room temperature for 20 hours to give the dimethylamino derivative (8).

To obtain the compounds of Formula I wherein n is two the bromide (7) shown in Chart II is reacted with sodium cyanide followed by hydrogenation and reductive alkylation by procedures well known in the art.

The amine compounds, (1) and (12) are prepared by the general method detailed in J. Org. Chem., 36(9): 1308 (1971) and depicted schematically in Chart III, wherein the substituent group Y is -NR₁R₂ or -C(=O)OCH₃. The substituted phenylacetonitrile (9) is reacted with the alpha-omega dibromobutyl compound (10) in the presence of base to produce the cycloalkyl nitrile, (11) which is catalytically reduced by the action of hydrogen over a noble metal catalyst to give the amines (1) and (12). Compounds (4) depicted in Chart II are prepared by reacting compounds (12) and (2) as generally described above.

As noted above, the compounds of the present invention wherein n is zero, and R₁ and R₂ are alkyl having from one to four carbon atoms are generically disclosed in EP-A 0 293 880. The compounds of the present invention as represented by general Formula I and their pharmaceutically acceptable acid salts have been found to be especially potent ACAT inhibitors, a finding which was unexpected and could not have been predicted in advance. This surprising superior activity of these compounds over analogs thereof was demonstrated in a chronic in vivo study in pure bred male and female beagle dogs. The average weight of the dogs was 6 kg, and each was fed a diet high in fatty acids for an 3- to ±0-week period, causing a twofold increase in the blood cholesterol levels of the dogs.

The dogs were divided into eight groups of six dogs each for a two-week study wherein the dogs were fed the same high content fatty acid diet either alone (two groups) or together with varying amounts of two test compounds (three groups for each test compound). The test compound was administered as bulk drug in a capsule in the high content fatty acid diet. The dogs were dosed once daily and fed from 8:00 AM to noon. Blood samples were drawn at 2:00 PM each day. The high content fatty acid diet consisted of 400 grams of dog chow containing 1% cholesterol, 5.5% lard, and 0.2% cholic acid. The two-week end results of the study are set forth in the following Table I wherein the data represents the percent change before and after treatment in each group.

**TABLE I**

| Dose (mg/kg) | Test Compound % Change | |
|---|---|---|
| | A | B |
| 0 | -1 | -1 |
| 1 | -9 | -12 |
| 3 | -14 | -28* |
| 10 | -11 | -39** |

| | | |
|---|---|---|
| * p = 0.045 by paired t-test. | | |
| ** p = 0.007 by paired t-test. | | |

Test compounds A and B have the following structures: 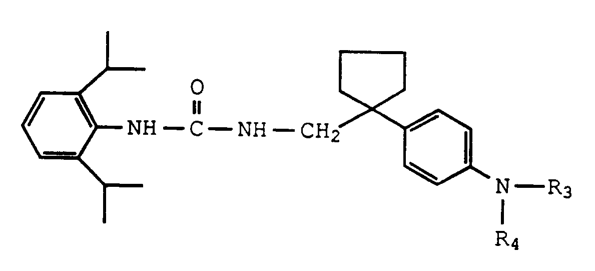
- Compound A:: R₃ = R₄ = H
- Compound B:: R₃ = R₄ = CH₃ (hydrochloride salt)

In therapeutic use as agents for the inhibition of intestinal absorption of cholesterol, or as hypolipidemic or hypocholesterolemic agents, the compounds utilized in the pharmaceutical method of this invention are administered to the patient at dosage levels of from 250 to 1000 mg per day. For a normal human adult of approximately 70 kg of body weight, this translates into a dosage of from 5 to 20 mg/kg of body weight per day. The specific dosages employed, however, may be varied depending upon the requirements of the patient, the severity of the condition being treated, and the activity of the compound being employed. The determination of optimum dosages for a particular situation is within the skill of the art.

For preparing pharmaceutical compositions from the compounds of this invention, inert, pharmaceutically acceptable carriers can be either solid or liquid. Solid form preparations include powders, tablets, dispersible granules, capsules, and cachets.

A solid carrier can be one or more substances which may also act as diluents, flavoring agents, solubilizers, lubricants, suspending agents, binders, or tablet disintegrating agents; it can also be an encapsulating material.

In powders, the carrier is a finely divided solid which is in a mixture with the finely divided active component. In tablets, the active compound is mixed with the carrier having the necessary binding properties in suitable proportions and compacted in the shape and size desired.

Powders and tablets preferably contain between about 5 to about 70% by weight of the active ingredient. Suitable carriers are magnesium carbonate, magnesium stearate, talc, lactose, sugar, pectin, dextrin, starch, tragacanth, methyl cellulose, sodium carboxymethyl cellulose, a low-melting wax, cocoa butter, and the like.

The term "preparation" is intended to include the formulation of the active compound with encapsulating material as a carrier providing a capsule in which the active component (with or without other carriers) is surrounded by a carrier, which is thus in association with it. In a similar manner, cachets are also included.

Tablets, powders, cachets, and capsules can be used as solid dosage forms suitable for oral administration.

Liquid form preparations include solutions suitable for oral administration, or suspensions and emulsions suitable for oral administration. Aqueous solutions for oral administration can be prepared by dissolving the active compound in water and adding suitable flavorants, coloring agents, stabilizers, and thickening agents as desired. Aqueous suspensions for oral use can be made by dispersing the finely divided active component in water together with a viscous material such as natural or synthetic gums, resins, methyl cellulose, sodium carboxymethyl cellulose, and other suspending agents known to the pharmaceutical formulation art.

Preferably, the pharmaceutical preparation is in unit dosage form. In such form, the preparation is divided into unit doses containing appropriate quantities of the active component. The unit dosage form can be a packaged preparation, the package containing discrete quantities of the preparation, for example, packeted tablets, capsules, and powders in vials or ampoules. The unit dosage form can also be a capsule, cachet, or tablet itself, or it can be the appropriate number of any of these packaged forms.

The following preparative examples are illustrative of the invention.

### Example 1

### Preparation of N-[2,6-bis(1-methylethyl)phenyl]-N'-[[1-(4-dimethylaminophenyl)cyclopentyl]methyl]urea

To a solution of 1-(4-dimethylaminophenyl)-cyclopentyl methyl amine (1.0 g, 0.0045 mole) in 30 ml of ethyl acetate, 2,6-bis(1-methylethyl)phenyl isocyanate (0.91 g, 0.0045 mole) is added and the reaction mixture is stirred at room temperature for 20 hours. Volatiles are removed under reduced pressure and the residue is crystallized from ethyl acetate-hexane yielding the title compound having a melting point of 132-133°C.

| Analysis calculated for C₂₇H₃₉N₃O | | | |
|---|---|---|---|
| | C = 76.92; | H = 9.32; | N = 9.97 |
| Found | C = 77.00; | H = 9.37; | N = 9.91 |

The starting [1-(4-dimethylaminophenyl)cyclopentyl]-methylamine was prepared as follows:

### 1-Cyano-1-(4-nitrophenyl)cyclopentane

To 79.2 g (1.65 m) of 50% sodium hydride suspended in 750 ml DMSO was added dropwise a mixture of 121.6 g (0.75 mole) p-nitrophenylacetonitrile and 161.7 ml (0.75 mole) 1,4-dibromobutane in 750 ml of a 50:50 mixture of DMSO, diethyl ether. The temperature was held between 25 to 30°C. The reaction mixture was stirred at room temperature overnight then cooled to 10°C. Thirty-eight milliliters of isopropanol was added followed by the cautious addition of 2.8 l of water. Air was bubbled through the black reaction mixture to remove most of the ether. Black solid was filtered and taken up with diethyl ether. The ether solution was washed two times with 2N HCl and two times with brine, dried over MgSO₄, and concentrated in vacuo. The resulting dark solid was extracted six times with boiling hexane. The hexane solution was concentrated to a small volume to yield 127.5 g of product, melting point 76 to 77°C.

### 1-Cyano-1-(4-dimethylaminophenyl)cyclopentane

In a pressure reactor was placed 127.5 g (0.59 mole) of 1-cyano-1-(4-nitrophenyl)cyclopentane and 1600 ml of methanol. One gram of 10% Pd/C was added to the reactor and charged with hydrogen. The reaction mixture was shaken at room temperature until the theoretical amount of hydrogen had been taken up. The reactor was vented and 100.5 g of 37% formaldehyde and 5 g of 10% Pd/C was added and shaken at room temperature for 16 hours. The reaction mixture was filtered and the filtrate concentrated in vacuo to give a sticky solid. The solid was taken up in ether and washed two times with a sodium bisulfite solution and two times with brine, dried over MgSO₄, and concentrated in vacuo. The product was purified by chromatography, using a gradient of 90% hexane: 10% ethyl acetate to 70% hexane: 30% ethyl acetate as eluant. There was obtained 94 g of the title compound, melting point 73 to 74°C.

| Analysis calculated for C₁₄H₁₈N₂ | | | |
|---|---|---|---|
| | C = 78.46; | H = 8.47; | N = 13.07 |
| Found | C = 78.48; | H = 8.50; | N = 13.03 |

### [1-(4-dimethylaminophenyl)cyclopentyl]methylamine

In a pressure reactor was placed 281.2 g (1.3 mole) of 1-cyano-1-(4-dimethylaminophenyl)cyclopentane, 3500 ml of methanol, 600 g of anhydrous ammonia, and 200 g of Raney nickel. Hydrogen was charged into the stirring reaction vessel. The reaction was stirred at room temperature until the theoretical amount of hydrogen had been taken up. The reaction mixture was filtered and the filtrate concentrated in vacuo to yield 284.4 g of the title compound, melting point 87 to 89°C.

| Analysis calculated for C₁₄H₂₂N₂ | | | |
|---|---|---|---|
| | C = 77.01; | H = 10.16; | N = 12.83 |
| Found | C = 76.81; | H = 10.13; | N = 12.63 |

### EXAMPLE 2

### N-[2,6-bis(1-methylethyl)phenyl]-N'-[[1-[3-[(dimethyl amino)methyl]phenyl]cyclopentyl]methyl] urea

### 1-Cyano-1-[3-(methylcarboxyl)phenyl]cyclopentane

The title compound is prepared in the same manner as 1-cyano-1-(4-nitrophenyl)cyclopentane in Example 1. Chromatography of the crude product in 10:1 hexane/ethyl acetate gave product as a clear, yellow oil.

| Analysis calculated for C₁₄H₁₅NO₂ | | | |
|---|---|---|---|
| | C = 73.34; | H = 6.59; | N = 6.11 |
| Found | C = 72.22; | H = 6.24; | N = 5.51 |

### [1-[3-(methylcarboxyl)phenyl]cyclopentyl]methylamine

In a pressure reactor was placed 22.8 g (0.099 mole) of 1-cyano-1-[3-(methylcarboxyl)phenyl]-cyclopentane, 200 mL of methanol, and 9.9 g of concentrated sulfuric acid. One gram of 20% Pd/C was added to the reactor, and it was charged with hydrogen. The reaction mixture was shaken at room temperature until the theoretical amount of hydrogen had been taken up. The reaction mixture was filtered and the filtrate concentrated in vacuo to a thick syrup. The syrup was taken up in 400 mL warm water and basified with 20% NaOH to approximately pH 13. The aqueous solution was extracted three times with diethyl ether. The combined organic extracts were washed with brine, dried over Na₂SO₄, and concentrated under vacuum to yield 19.9 g of a clear, colorless oil.

### 3-[1-[[[[[2,6-bis(1-methylethyl)phenyl]amino]carbonyl] amino]methyl]cyclopentyl]benzoic acid, methyl ester

In 500 mL of ethyl acetate were dissolved 19.9 g (0.085 mole) of [1-[3-(methylcarboxyl)phenyl]cyclopentyl]methyl amine and 17.3 g (0.085 mole) of [2,6-bis(1-methylethyl)phenyl]isocyante. The reaction mixture was stirred at room temperature for 20 hours. The mixture was concentrated to dryness in vacuo. The resulting residue was suspended in hexane and collected. The filter cake was oven-dried affording 26.4 g of a fluffy white solid, m.p. 149-151°C.

| Analysis calculated for C₂₇H₃₆N₂O₃ | | | |
|---|---|---|---|
| | C = 74.28; | H = 8.31; | N = 6.42 |
| Found | C = 74.36; | H = 8.38; | N = 6.17 |

### 3-[1-[[[[[2,6-bis(1-methylethyl)phenyl]amino]carbonyl] amino]methyl]cyclopentyl]benzoic acid

In 800 mL methanol were dissolved the following: 24.0 g (0.052 mole) of 3[1-[[[[[2,6-bis(1-methylethyl)-phenyl]amino]carbonyl]amino]methyl]cyclopentyl]benzoic acid, methyl ester and 53 mL (0.053 mole) of 1N NaOH. The mixture was heated to reflux for 20 hours, then cooled to room temperature and concentrated in vacuo. The residue was taken up in 600 mL of hot water and acidified with concentrated hydrochloric acid to approximately pH 1. The resulting suspension was chilled several hours, and the solid was collected and oven-dried. Obtained were 23.4 g of a fluffy white solid, m.p. 209-211°C.

| Analysis calculated for C₂₆H₃₄N₂O₃ | | | |
|---|---|---|---|
| | C = 73.90; | H = 8.11; | N = 6.63 |
| Found | C = 73.37; | H = 7.93; | N = 6.35 |

### N-[2,6-bis(1-methylethyl)phenyl]-N'-[[1-[3-(hydroxy methyl)phenyl[cyclopentyl]methyl] urea

3-[1-[[[[[2,6-bis(1-methylethyl)phenyl]amino]-carbonyl]amino]methyl]cyclopentyl]benzoic acid (50.3 g, 0.119 mole) was suspended in 750 mL of tetrahydrofuran. Trimethylborate (29.7 mL, 0.262 mole) was added and the mixture was heated to reflux under nitrogen. The heat source was removed and 59.8 mL (0.631 mole) of borane methyl sulfide was added at a rate so as to maintain reflux. After complete addition, the mixture was heated to reflux an additional 4 hours, then stirred at room temperature for 20 hours. The reaction was then cooled to 5°C and quenched by slowly adding 240 mL of methanol. The reaction mixture was concentrated to give a brown gum, which was chromatographed in 1:1 hexane/ethyl acetate to yield 42.2 g of a white solid, m.p. 152-158°C.

| Analysis calculated for C₂₆H₃₆N₂O₂ | | | |
|---|---|---|---|
| | C = 76.43; | H = 8.88; | N = 6.86 |
| Found | C = 76.48; | H = 8.89; | N = 6.73 |

### N-[2,6-bis(1-methylethyl)phenyl]-N'-[[1-[3-(bromo methyl)phenyl]cyclopentyl]methyl] urea

Phosphorus tribromide (0.12 g, 0.0004 mole) was dissolved in 50 mL toluene under nitrogen. Pyridine (0.025 g, 0.0003 mole) was added and the mixture was cooled to 0°C. N-[2,6-bis(1-methylethyl)phenyl]-N'-[1-[3-(hydroxymethyl)phenyl]cyclopentyl]methyl] urea (0.05 g, 0.0012 mole) was dissolved in 50 mL toluene and added slowly, holding temperature at 0° to 2°C. The reaction was allowed to warm gradually to room temperature over 5 hours, then stirred at room temperature an additional 67 hours. Volatiles were removed under reduced pressure and the gummy residue was purified by chromatography in 4:1 hexane/ethyl acetate. Obtained was 0.51 g of a white solid, m.p. 178-181°C.

| Analysis calculated for C₂₆H₃₅BrN₂O | | | | |
|---|---|---|---|---|
| | C = 66.23; | H = 7.48; | N = 5.94; | Br = 16.95 |
| Found | C = 66.18; | H = 7.55; | N = 5.85; | Br = 16.95 |

### N-[2,6-bis(1-methylethyl)phenyl]-N'-[[1-[3-[(dimethyl amino)methyl]phenyl]cyclopentyl]methyl] urea

A round bottom flask was charged with 250 mL of a saturated solution of dimethylamine in methanol. N-[2,6-bis(1-methylethyl)phenyl]-N'-[[1-[3-(bromomethyl)phenyl]cyclopentyl]methyl]urea (25.5 g, 0.054 mole) was suspended in 250 mL methanol and added to the dimethylamine solution. The reaction mixture was stirred at room temperature for 20 hours, then concentrated in vacuo. The residue was taken up in 250 mL 2N NaOH and stirred with 250 mL chloroform for 0.5 hour. The layers were separated; the aqueous portion was extracted two times with chloroform. The combined organic extracts were washed with water and then with brine. The solution was dried over Na₂SO₄ and concentrated in vacuo. The residue was triturated under hexane and collected. The filtrates were concentrated and the solid collected three times. The combined filter cakes were oven-dried to a constant weight. A fine, white solid (21.9 g) was obtained, m.p. 111-120°C.

| Analysis calculated for C₂₈H₄₁N₃O·0.05 H₂O | | | |
|---|---|---|---|
| | C = 75.63; | H = 9.52; | N = 9.45 |
| Found | C = 75.88; | H = 9.22; | N = 8.81 |

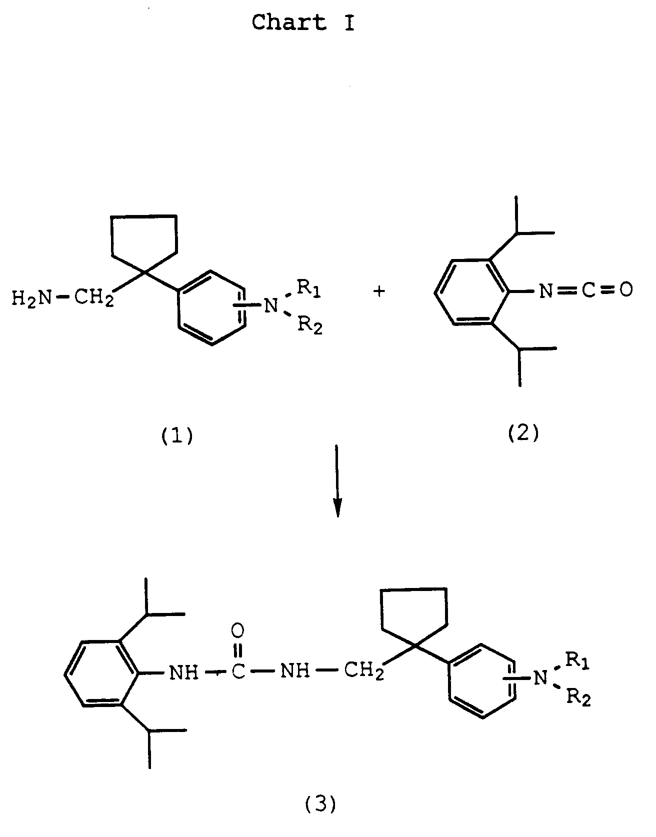

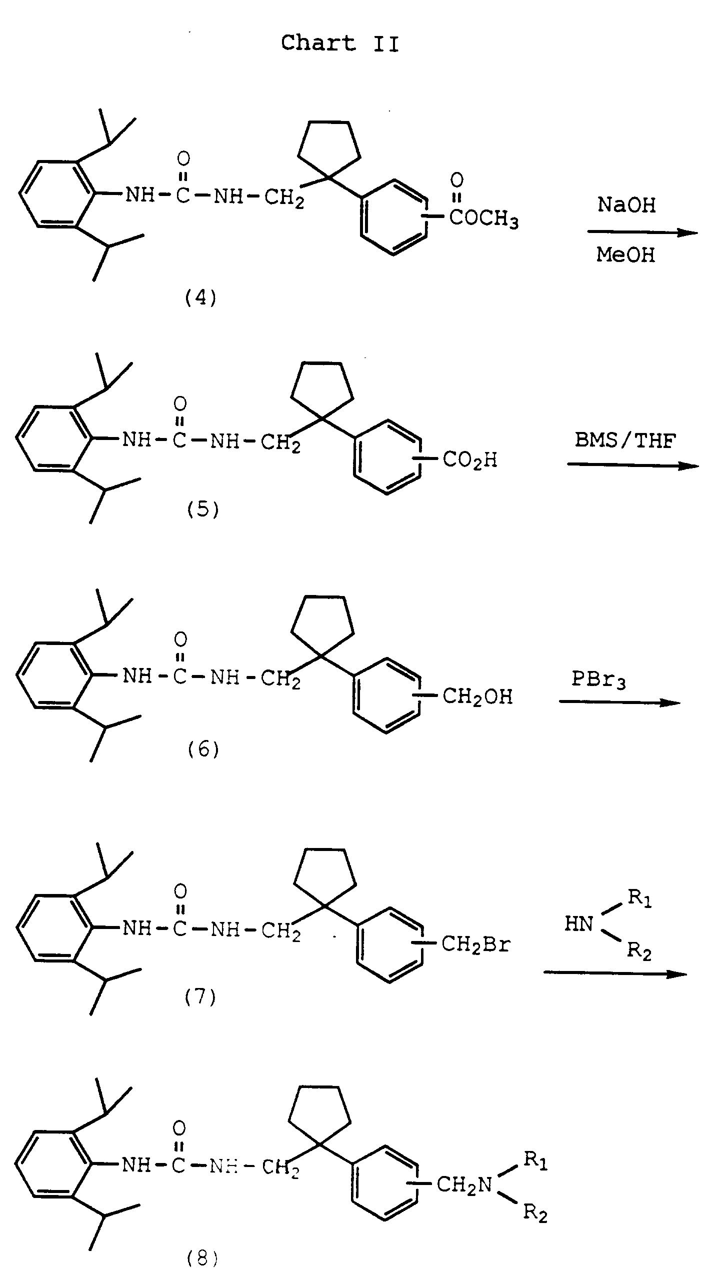

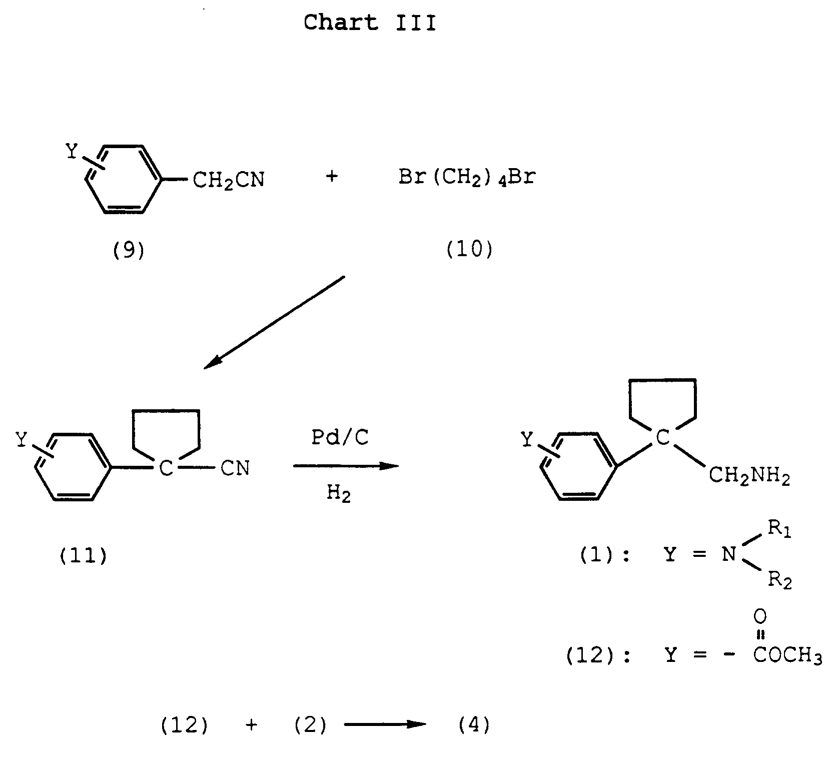

## Claims

1. A compound of the formula 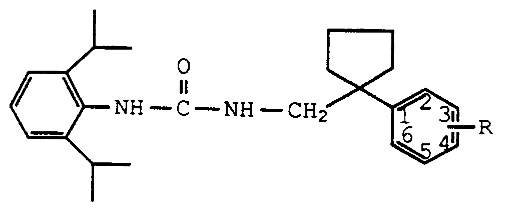 wherein R is attached to the 3- or 4-position of the phenyl ring and is the group: 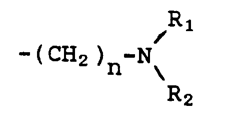 wherein n is zero, one, or two, and wherein each of R₁ and R₂ is a straight or branched lower alkyl group having from one to four carbon atoms or alkyl having from one to four carbon atoms wherein the terminal carbon atom is substituted with an OR₃ group wherein R₃ is hydrogen or alkyl having from one to four carbon atoms, and pharmaceutically acceptable acid salts thereof.

2. A compound of Claim 2 wherein n is zero.

3. A compound of Claim 2 which is N-[2,6-bis(1-methylethyl)phenyl]-N'-[[1-(4-dimethylaminophenyl)cyclopentyl]methyl]urea.

4. A compound of Claim 3 which is N-[2,6-bis(1-methylethyl)phenyl]-N'-[[1-(4-dimethylaminophenyl)cyclopentyl]methyl]urea hydrochloride.

5. A compound of Claim 2 which is N-[2,6-bis(1-methylethyl)phenyl]-N'-[[1-(4-diethylaminophenyl)cyclopentyl]methyl]urea.

6. A compound of Claim 2 which is N-[2,6-bis(1-methylethyl)phenyl]-N'-[[1-(4-diisopropylaminophenyl)cyclopentyl]methyl]urea.

7. A compound of Claim 2 which is N-[2,6-bis(1-methylethyl)phenyl]-N'-[[1-(4-di-n-butylaminophenyl)cyclopentyl]methyl]urea.

8. A compound of Claim 1 wherein n is one.

9. A compound of Claim 8 which is N-[2,6-bis(1-methylethyl)phenyl]-N'-[[1-[3-[(dimethylamino)-methyl]phenyl]cyclopentyl]methyl]urea.

10. A compound of Claim 8 which is N-[2,6-bis(1-methylethyl)phenyl]-N'-[[1-[4-[(dimethylamino)-methyl]phenyl]cyclopentyl]methyl]urea.

11. A compound of Claim 8 which is N-[2,6-bis(1-methylethyl)phenyl]-N'-[[1-[3-[(diethylamino)-methyl]phenyl]cyclopentyl]methyl]urea.

## Patentansprüche

1. Verbindung der Formel 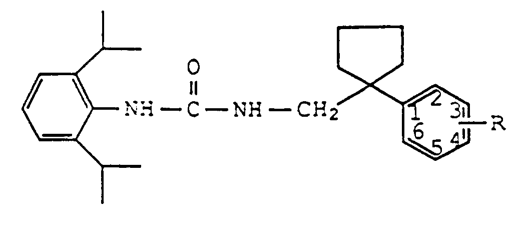 worin R an die 3- oder 4-Stellung des Phenylringes gebunden ist und eine Gruppe der Formel 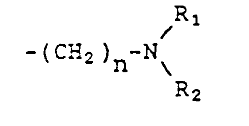 darstellt, worin
n Null, 1 oder 2; und die Gruppen
R₁ und R₂, jede für sich, eine geradkettige oder verzweigte Niederalkylgruppe mit 1 bis 4 Kohlenstoffatomen oder Alkyl mit 1 bis 4 Kohlenstoffatomem, worin das endständige Kohlenstoffatom mit einer OR₃-Gruppe substituiert ist, wobei R₃ für Wasserstoff oder Alkyl mit 1 bis 4 Kohlenstoffatomen steht,
bedeuten, und deren pharmazeutisch annehmbaren Säureadditionssalze.

2. Verbindung nach Anspruch 1, worin n Null bedeutet.

3. N-[2,6-Bis(1-methylethyl)phenyl]-N'-[{1-(4-dimethylaminophenyl)cyclopentyl}methyl]harnstoff als Verbindung nach Anspruch 2.

4. N-[2,6-Bis(1-methylethyl)phenyl]-N'-[{1-(4-dimethylaminophenyl)cyclopentyl}methyl]harnstoff-hydrochlorid als Verbindung nach Anspruch 3.

5. N-[2,6-Bis(1-methylethyl)phenyl]-N'-[{1-(4-diethylaminophenyl)cyclopentyl}methyl]harnstoff als Verbindung nach Anspruch 2.

6. N-[2,6-Bis(1-methylethyl)phenyl]-N'-[{1-(4-diisopropylaminophenyl)cyclopentyl}methyl]harnstoff als Verbinung nach Anspruch 2.

7. N-[2,6-Bis(1-methylethyl)phenyl]-N'-[{1-(4-di-n-butylaminophenyl)cyclopentyl}methyl]harnstoff als Verbinung nach Anspruch 2.

8. Verbindung nach Anspruch 1, worin n für 1 steht.

9. N-[2,6-Bis(1-methylethyl)phenyl]-N'-[{1-[3-{(dimethylamino)methyl}phenyl]cyclopentyl}methyl]harnstoff als Verbindung nach Anspruch 8.

10. N-[2,6-Bis(1-methylethyl)phenyl)-N'-[{1-[4-{(dimethylamino)methyl}phenyl]cyclopentyl}methyl]harnstoff als Verbindung nach Anspruch 8.

11. N-[2,6-Bis(1-methylethyl)phenyl]-N'-[{1-[3{(diethylamino)-methyl}phenyl]cyclopentyl}methyl]harnstoff als Verbindung nach Anspruch 8.

## Revendications

1. Un composé de formule: 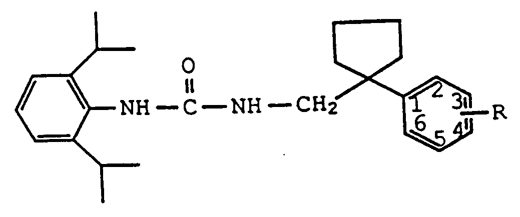 dans laquelle:
R est lié à la position 3- ou 4- du cycle phényle, et représente le groupe: 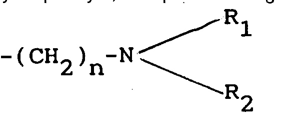
dans lequel:
n est égal à 0, 1 ou 2; et
R₁ et R₂ sont chacun un radical alkyle inférieur, linéaire ou ramifié, présentant de 1 à 4 atomes de carbone, ou radical alkyle ayant de 1 à 4 atomes de carbone dans lequel l'atome de carbone terminal est substitué avec un groupe OR₃ dans lequel R₃ est un atome d'hydrogène ou un radical alkyle ayant de 1 à 4 atomes de carbone,
et les sels d'acide pharmaceutiquement acceptables de ce dernier.

2. Un composé selon la revendication 1, dans lequel n est égal à zéro.

3. Un composé selon la revendication 2, qui est la N-[2,6-bis(1-méthyléthyl)phényl]-N'-[[1-(4-diméthylaminophényl)cyclopentyl]méthyl]urée.

4. Un composé selon la revendication 3, qui est le chlorhydrate de N-[2,6-bis(1-méthyléthyl)phényl]-N'-[[1-(4-diméthylaminophényl)cyclopentyl]méthyl]urée.

5. Un composé selon la revendication 2, qui est la N-[2,6-bis(1-méthyléthyl)phényl]-N'-[[1-(4-diéthylaminophényl)cyclopentyl]méthyl]urée.

6. Un composé selon la revendication 2, qui est la N-[2,6-bis(1-méthyléthyl)phényl]-N'-[[1-(4-diisopropylaminophényl)cyclopentyl]méthyl]urée.

7. Un composé selon la revendication 2, qui est la N-[2,6-bis(1-méthyléthyl)phényl]-N'-[[1-(4-di-n-butylaminophényl)-cyclopentyl]méthyl]urée.

8. Un composé selon la revendication 1, dans lequel n est égal à un.

9. Un composé selon la revendication 8, qui est la N-[2,6-bis(1-méthyléthyl)phényl]-N'-[[1-[3-[(diméthylamino)-méthyl]phényl]cyclopentyl]méthyl]urée.

10. Un composé selon la revendication 8, qui est la N-[2,6-bis(1-méthyléthyl)phényl]-N'-[[1-[4-[(diméthylamino)méthyl]phényl]cyclopentyl]méthyl]urée.

11. Un composé selon la revendication 8, qui est la N-[2,6-bis(1-méthyléthyl)phényl]-N'-[[1-[3-[(diéthylamino)-méthyl]phényl]cyclopentyl]méthyl]urée.
